# EUROPEAN PATENT APPLICATION

(11) **EP 3 508 840 A1**
(43) Date of publication of application: **10.07.2019**
(21) Application number: 17846497.0
(22) Date of filing: 29.08.2017
(51) Int. Cl.: G01N 27/26, A61M 1/16, G01N 1/00

(54) **STANDARD REAGENT KIT FOR DIALYSIS FLUID ANALYSIS, AND AQUEOUS SOLUTION FOR STANDARD REAGENT, DIALYSIS FLUID, AND ARTIFICIAL KIDNEY FLUID REPLENISHMENT**

(30) Priority: 30.08.2016 JP 2016168127; 30.08.2016 JP 2016168128; 28.08.2017 JP 2017163217
(71) Applicant: Nipro Corporation, Osaka-shi, Osaka 531-8510 (JP)
(72) Inventor: INOUE, Hiroyuki, Osaka-shi Osaka 531-8510 (JP); SHIROUCHI, Yutaka, Osaka-shi Osaka 531-8510 (JP); HIRATSUKA, Hiroyuki, Mie 5140008 (JP); FUKUSHIMA, Hiroshi, Osaka-shi Osaka 531-8510 (JP); NISHIYAMA, Kaname, Osaka-shi Osaka 531-8510 (JP)
(74) Representative: Vossius & Partner Patentanwälte Rechtsanwälte mbB
(86) International application number: PCT/JP2017/030962
(87) International publication number: WO 2018/043492

(57) **Abstract**

The present invention relates to a standard reagent kit for analysis of a dialysis fluid comprising of (1) a vial A filled with an aqueous solution A comprising an electrolyte component capable of reacting with carbonate ion to form a precipitate, and (2) a vial B filled with an aqueous solution B comprising at least either one of bicarbonate ion and carbonate ion, wherein at least the vial B is a water-repellent resin vial, and provides a standard reagent for analysis of a dialysis fluid, wherein precipitation is prevented for a long period of time, a change of a content of bicarbonate ion is controlled, a complicated procedure is not required for preparation thereof, handling thereof is easy, and checking of a dialysis fluid after preparation thereof can be performed easily and accurately at medical treatment site.

## Description

### TECHNICAL FIELD

The present invention relates to a standard reagent kit for analysis of a dialysis fluid, in particular a standard reagent kit allowing easy handling and being excellent in stability with time.

The present invention also relates to an aqueous solution comprising at least either one of bicarbonate ion or carbonate ion and used for a standard reagent for analysis of a dialysis fluid, a dialysis fluid and a substitution fluid for an artificial kidney, in particular to an aqueous solution comprising at least either one of bicarbonate ion or carbonate ion and being excellent in stability with time.

### BACKGROUND ART

In the case where a patient being subject to hypofunction of a kidney undergoes hemodialysis, blood of the patient is purified in an artificial kidney. Generally, in the artificial kidney, a dialysis fluid is subjected to perfusion and a waste product in blood is transferred to the dialysis fluid side via a dialyzer. In recent years, in order to reduce a burden of a patient, a sodium bicarbonate-containing dialysis fluid including a reduced amount of acetic acid has been used widely as a dialysis fluid instead of a conventional acetic acid dialysis fluid.

A sodium bicarbonate-containing dialysis fluid is not suitable as one formulation since an insoluble compound is generated due to reaction of electrolyte components (e.g., sodium chloride, potassium chloride, calcium chloride, magnesium chloride) with bicarbonate ion, and therefore, a dialysis fluid is usually prepared by pouring a concentrated solution of a preparation including electrolyte components and a pH regulator (e.g., acetic acid) (hereinafter referred to as "formulation A") and a concentrated solution of a preparation including sodium bicarbonate (hereinafter referred to as "formulation B") together with water into a dialysis fluid feeding device and mixing and diluting a mixture. With respect to these concentrated solution A and solution B, there is a case where these are prepared by dissolving respective commercially available powder formulations in water, or a case where one commercially available as a solution (aqueous solution) from the first is used.

A prepared dialysis fluid is used usually after confirming that a concentration of an electrolyte, a pH value, an osmotic pressure and the like are within proper ranges before use. Specifically there are points to be noted at the time of preparation of the dialysis fluid such as 1) measuring a concentration of an electrolyte of a dialysis fluid before use and confirming that it is a proper value, 2) confirming that a pH value of a dialysis fluid is within a range of from 7.2 to 7.4 before use since it may vary somewhat due to an influence of dilution water, and 3) when measuring an osmotic pressure of a dialysis fluid, measuring an osmotic pressure of a physiological salt solution and correcting an actually measured value. Further, manufacturers of a dialysis fluid feeding apparatus raise the matters to be noted and alarms such as 1) confirming that before starting therapy, an actual concentration of a dialysis fluid is as formulated using a test apparatus such as an osmometer, a conductance meter or a flame photometer, and 2) confirming using a test paper and a test chemical that after completion of depuration, a chemical for sterilization or acid cleaning does not remain in a fluid circuit. In any cases, it is an important matter to confirm a concentration of a prepared dialysis fluid.

The above-mentioned confirmation of a concentration of a prepared dialysis fluid is performed by means of flame photometric determination, an atomic absorption method, an ion-selective electrode method (ISE method) and the like. For calibration of measuring apparatuses used therefor, calibration solutions prepared based on a working reference standard substance are used. For example, Patent Document 1 discloses a dedicated calibration solution being capable of compensating accuracy of a concentration of a dialysis fluid in an electrolysis measuring apparatus using a principle of an electrode method.

### PRIOR ART DOCUMENTS

### Patent Document

Patent Document 1: JP 2010-271102 A

### SUMMARY OF THE INVENTION

### PROBLEM TO BE SOLVED BY THE INVENTION

However, calibration solutions for measuring apparatuses for checking a prepared dialysis fluid are in such a state that electrolyte components (components of formulation A) such as sodium chloride, potassium chloride, calcium chloride or magnesium chloride and sodium bicarbonate (a component of formulation B) coexist, and therefore, a sediment (CaCO₃, MgCO₃ or the like) is precipitated with a lapse of time or a content of bicarbonate ion varies easily, thus causing concern about stability with time. In a calibration solution disclosed in Patent Document 1, 2.0 ml each of a prepared calibration solution is measured and poured in a glass ampule and the ampule is hermetically sealed. However, with respect to precipitation of a sediment and a content of bicarbonate ion, sufficient storage stability is not shown.

Further, in a calibration solution for checking a prepared dialysis fluid, it is demanded that evaluation of a dialysis fluid can be carried out without taking complicated procedures such as dissolution of a solid preparation, dilution of a concentrated solution and the like before use thereof.

Thus, an object of the present invention is to provide a standard reagent for analysis of a dialysis fluid, wherein precipitation is prevented for a long period of time, a change of a content of bicarbonate ion is controlled, a complicated procedure is not required for preparation thereof, handling thereof is easy, and checking of a dialysis fluid after preparation thereof can be performed easily and accurately at medical treatment site.

### MEANS TO SOLVE THE PROBLEM

The inventors of the present invention have found that by filling an electrolyte component capable of reacting with carbonate ion to form a precipitate and bicarbonate ion and carbonate ion in separate vials to produce a standard reagent kit for analysis of a dialysis fluid and in this kit, using a water-repellent resin vial as the vial filled with an aqueous solution comprising at least bicarbonate ion and carbonate ion, precipitation of a sediment can be prevented for a long period of time and a standard reagent for analysis of a dialysis fluid can be prepared by very easy and secure mixing, and have completed a first embodiment of the present invention.

Further, the inventors of the present invention have found that by filling an electrolyte component capable of reacting with carbonate ion to form a precipitate, and bicarbonate and carbonate ion in separate vials, even in a case of a long term storage, precipitation of a sediment and a change of a content of bicarbonate ion can be controlled, and by communicating the both vials using a transfer needle at time of use for mixing, the mixing can be carried out very easily, and have completed a second embodiment of the present invention.

In this standard reagent kit, while precipitation of a sediment can be prevented and a change of a content of bicarbonate ion can be controlled as compared with a prior art, there is a case where in the vial B filled with an aqueous solution of sodium bicarbonate, carbon dioxide is generated with a lapse of time and is released into a gaseous phase and at the same time, there is a case where a concentration of bicarbonate ion and a pH value in the content change, which causes a concern such that a pH value and a concentration of bicarbonate ion of an obtained standard reagent will be out of a desired range. Therefore, there is still a room for improvement with respect to a change of a concentration of bicarbonate ion and a pH value.

Meanwhile, also with respect to a formulation B (preparation comprising sodium bicarbonate) to be used for a sodium bicarbonate-containing dialysis fluid, in a case of commercially available solutions (concentrated solutions), there is a concern about variation with time of a concentration of bicarbonate ion and a pH value.

Further, variation with time of a bicarbonate ion concentration and a pH value of an aqueous solution of sodium bicarbonate is a problem which may arise also in a substitution fluid for an artificial kidney. Namely, in hemocatharsis by using an artificial kidney, there is a case of using a substitution fluid for an artificial kidney having the substantially same composition as that of a sodium bicarbonate-containing dialysis fluid after mixing. Such a substitution fluid for an artificial kidney is generally sold in such a state that an aqueous solution including saccharum and electrolyte components (corresponding to components form formulation A in the dialysis fluid. There is a case where thses are called "formulation B" in substitution fluids for an artificial kidney which are generally available on the market. Herein these are called "components from formulation A" as a matter of convenience) and an aqueous solution including sodium bicarbonate (corresponding to component of formulation B in the dialysis fluid There is a case where this is called "formulation A" in substitution fluids for an artificial kidney which are generally available on the market. Herein this is called "component of formulation B" as a matter of convenience) are filled in each chamber of a double bag separated into two chambers by a partition wall. In this case, carbon dioxide gas is generated in the chamber filled with the aqueous solution including sodium bicarbonate, and there is a concern about variation with time of a component concentration and a pH value.

Therefore, in order to prevent variation with time of the formulation B for a sodium bicarbonate-containing dialysis fluid and the component of formulation B for a substitution fluid for an artificial kidney which are solutions, for such solutions on the market, countermeasures have been taken such that a whole bag filled with the formulation B for a sodium bicarbonate-containing dialysis fluid or a whole double bag filled with a substitution fluid for an artificial kidney is sealed with a gas barrier package, and further a carbon dioxide gas-generating deoxidant is filled in a sealed space.

Thus, an object of the present invention is to provide aqueous solutions for a standard reagent, a dialysis fluid and a substitution fluid for an artificial kidney, which include at least either one of bicarbonate ion or carbonate ion, are excellent in stability and assure that variation with time of a bicarbonate ion concentration and a pH value is prevented more, and further, the inventors of the present invention have found that in aqueous solutions for a standard reagent, a dialysis fluid and a substitution fluid for an artificial kidney, which include at least either one of bicarbonate ion or carbonate ion, variation with time of a bicarbonate ion concentration and a pH value which results from releasing of a carbon dioxide gas can be inhibited by adjusting pH values of the solutions to be 8.6 to 10.5, and have completed a third embodiment of the present invention.

Namely, the first embodiment of the present invention relates to:
[1] a standard reagent kit for analysis of a dialysis fluid comprising:
   (1) a vial A filled with an aqueous solution A comprising an electrolyte component capable of reacting with carbonate ion to form a precipitate, and
   (2) a vial B filled with an aqueous solution B comprising at least either one of bicarbonate ion or carbonate ion,
   wherein at least the vial B is a water-repellent resin vial,
[2] the standard reagent kit of the above [1], wherein a contact angle of water on a surface of the water-repellent resin vial is 90 degrees or more, preferably 100 degrees or more, more preferably 110 degrees or more, further preferably 120 degrees or more, particularly preferably 130 degrees or more, still further preferably 140 degrees or more, most preferably 150 degrees or more,
[3] the standard reagent kit of the above [1] or [2], wherein a total light transmittance of the water-repellent resin vial is 85% or more, preferably 90% or more,
[4] the standard reagent kit of any of the above [1] to [3], wherein the water-repellent resin vial is formed from a cycloolefin polymer (COP),
[5] the standard reagent kit of any of the above [1] to [4], wherein a pH value of the solution B is 8.9 or more, preferably 9.2 or more, more preferably 9.3 or more, and
[6] the standard reagent kit of any of the above [1] to [5], wherein the standard reagent for analysis of a dialysis fluid is prepared by pouring either one of the solution A or the solution B filled in the water-repellent resin vial into a vial filled with another solution and subjecting the solutions to mixing,
the second embodiment of the present invention relates to:
[1] a standard reagent kit for analysis of a dialysis fluid comprising:
   (1) a vial A filled with an aqueous solution A comprising an electrolyte component capable of reacting with carbonate ion to form a precipitate,
   (2) a vial B filled with an aqueous solution B comprising at least either one of bicarbonate ion and carbonate ion, and
   (3) a transfer needle for mixing a content of the vial A and a content of the vial B,
[2] the standard reagent kit of the above [1], wherein a pH value of the solution B is from 8.6 to 10.5, preferably from 9.0 to 10.2, more preferably from 9.2 to 10.1, and
[3] the standard reagent kit of the above [1] or [2], wherein the electrolyte component capable of reacting with carbonate ion to form a precipitate comprise calcium ion and magnesium ion, and
the third embodiment of the present invention relates to:
[1] an aqueous solution comprising at least either one of bicarbonate ion and carbonate ion and having a pH value of from 8.6 to 10.5, preferably from 9.0 to 10.2, more preferably from 9.2 to 10.1, wherein the aqueous solution is combined with an aqueous solution comprising an electrolyte component capable of reacting with carbonate ion to form a precipitate and is used as a standard reagent for analysis of a dialysis fluid,
[2] the aqueous solution of the above [1] filled in a vial,
[3] a standard reagent for analysis of a dialysis fluid, the standard reagent being prepared by combining an aqueous solution comprising an electrolyte component capable of reacting with carbonate ion to form a precipitate with the aqueous solution of the above [1] or [2],
[4] an aqueous solution comprising at least either one of bicarbonate ion and carbonate ion and having a pH value of from 8.6 to 10.5, preferably from 9.0 to 10.2, more preferably from 9.2 to 10.1, wherein the aqueous solution is combined with an aqueous solution comprising an electrolyte component capable of reacting with carbonate ion to form a precipitate and is used as a dialysis fluid,
[5] an aqueous solution comprising at least either one of bicarbonate ion and carbonate ion and having a pH value of from 8.6 to 10.5, preferably from 9.0 to 10.2, more preferably from 9.2 to 10.1, wherein the aqueous solution is combined with an aqueous solution comprising an electrolyte component capable of reacting with carbonate ion to form a precipitate and is used as a substitution fluid for an artificial kidney,
[6] the aqueous solution of the above [5] filled in one chamber of a multi-chamber vessel having at least two chambers which are separated by an isolation means and can be communicated with each other, and
[7] a substitution fluid for an artificial kidney prepared by combining an aqueous solution comprising an electrolyte component capable of reacting with carbonate ion to form a precipitate with the aqueous solution of the above [5] or [6].

### EFFECTS OF THE INVENTION

According to the first embodiment of the present invention, an aqueous solution comprising electrolyte components reacting with carbonate ion to form a precipitate and an aqueous solution comprising at least either one of bicarbonate ion and carbonate ion are filled in separate vials to make a kit comprising these vials, and in this kit, the vial filled with the aqueous solution comprising at least either one of bicarbonate ion and carbonate ion is a water-repellent resin vial, thereby enabling sedimentation of a precipitate to be prevented for a long period of time and allowing a standard reagent for analysis of a dialysis fluid to be prepared by mixing the aqueous solutions very simply and surely.

According to the second embodiment of the present invention, an aqueous solution comprising electrolyte components reacting with carbonate ion to form a precipitate and an aqueous solution comprising at least either one of bicarbonate ion and carbonate ion are filled in separate vials, and a kit comprising these two vials and a transfer needle for mixing the aqueous solutions at time of use is formed, thereby being capable of providing a standard reagent for analysis of a dialysis fluid which requires no complicated procedures for preparation thereof, assures easy handling, prevents sedimentation of a precipitate for a long period of time, and inhibits variation of a content of bicarbonate ion.

According to the third embodiment of the present invention, an aqueous solution being excellent in stability and used as a standard reagent for analysis of a dialysis fluid, an aqueous solution being excellent in stability and used as a dialysis fluid and an aqueous solution being excellent in stability and used as a substitution fluid for an artificial kidney can be provided by adjusting pH values of the aqueous solutions to be within a predetermined range, thereby preventing variation with time of a bicarbonate ion concentration and a pH value of an aqueous solution comprising at least either one of bicarbonate ion and carbonate ion. Thus, for example, for an aqueous solution of a standard reagent for analysis of a dialysis fluid, an unprecedented standard reagent for analysis of a dialysis fluid can be provided, and for a concentrated aqueous solution for a dialysis fluid and an aqueous solution for a substitution fluid for an artificial kidney, countermeasures which have been taken such as gas barrier packaging and packing of a carbon dioxide gas-generating deoxidant are not necessary.

### EMBODIMENT FOR CARRYING OUT THE INVENTION

### [Standard reagent kit for analysis of a dialysis fluid according to first embodiment]

The standard reagent kit for analysis of a dialysis fluid according to the first embodiment of the present invention comprises (1) a vial A filled with an aqueous solution A comprising an electrolyte component capable of reacting with carbonate ion to form a precipitate, and (2) a vial B filled with an aqueous solution B comprising at least either one of bicarbonate ion and carbonate ion, wherein at least the vial B is a water-repellent resin vial. In the standard reagent kit for analysis of a dialysis fluid according to the first embodiment of the present invention, sedimentation of a precipitate is prevented at least for 6 months or more, preferably for one year or more, and a bicarbonate ion content and a pH value hardly vary.

Herein, a "water-repellent resin vial" is not limited particularly as long as an inner surface of the vial repels water, and in the case where an water-soluble liquid filled in the vial is poured out from the vial, the water-soluble liquid does not remain in the vial, or even if it remains in the vial, its amount is very small (for example, a remaining amount is not more than 1% of the filled amount). Specifically, a vial having a contact angle of water on a surface of the vial of 90 degrees or more is used preferably. The contact angle of water on a surface (especially an inner surface) of the water-repellent resin vial is usually preferably 90 degrees or more, more preferably 100 degrees or more, further preferably 110 degrees or more, particularly preferably 120 degrees or more, further preferably 130 degrees or more, further preferably 140 degrees or more, most preferably 150 degrees or more. The water-repellent resin vial may be one formed using a water-repellent resin being capable of exhibiting a contact angle of water as mentioned above as a material thereof, or may be one produced by subjecting a surface (especially inner surface) of a vial formed from other material to known water-repelling treatment. In the present invention, the contact angle of water can be measured by a sessile drop method in accordance with JIS-R3257.

Examples of the water-repellent resin for forming the water-repellent resin vial include a cycloolefin polymer (COP), a cycloolefin copolymer (COC), polytetrafluoroethylene (PTFE), polypropylene (PP), polyethylene (PE) and the like. Among these, a cycloolefin polymer (COP) and a cycloolefin copolymer (COC) are suitable.

A total light transmittance of the water-repellent resin vial is preferably 85% or more, more preferably 90% or more. In the present invention, the total light transmittance can be measured in accordance with JIS-K7361-1 referred to in JIS-K7375.

### <Solution A>

The solution A is an aqueous solution comprising an electrolyte component capable of reacting with carbonate ion to form a precipitate. Examples of the electrolyte component capable of reacting with carbonate ion to form a precipitate include calcium ion, magnesium ion and the like. These electrolyte components can be contained in the solution A as calcium chloride, magnesium chloride and the like. In the case of calcium chloride and magnesium chloride are used for the standard reagent, these are contained in the solution A as electrolytes. It is a matter of course that in the first embodiment of the present invention, carbonate ion and bicarbonate ion are not contained in the solution A.

In addition, it is possible that the solution A contains other electrolytes such as sodium chloride, potassium chloride and sodium acetate and an organic salt. Further, hydrochloric acid and an organic acid can be used as a pH regulator, and it is preferable to use, as an organic acid, acetic acid, citric acid, oxalic acid, tartaric acid, maleic acid, ascorbic acid, oxalacetic acid, gluconic acid, isocitric acid, malic acid, and/or citric acid. Further it is preferable to mix glucose in the solution A.

Examples of usable calcium chloride include calcium chloride dihydrate, calcium chloride monohydrate, calcium chloride anhydride and the like. Examples of magnesium chloride include magnesium chloride hexahydrate and the like.

Anhydrous sodium acetate, sodium acetate trihydrate and the like can be used preferably as sodium acetate. Further, acetic acid (glacial acetic acid) and sodium hydroxide can be added, resulting in addition of sodium acetate.

An amount of the solution A is preferably 3 mL or more, more preferably 5 mL or more from the viewpoint of controlling a filling amount accurately. Further, the amount of the solution A is preferably 20 mL or less, more preferably 10 mL or less from the viewpoint of handling such as mixing operation.

A pH value of the solution A is preferably 2.5 or more, more preferably 3 or more in consideration of an influence on corrosion of a production apparatus and the like. Further the pH value of the solution A is preferably 7 or less, more preferably 6 or less. If the pH value of the solution A is too high, a pH value of the solution B cannot help being set to be lower in order to maintain a pH value of the standard reagent obtained by mixing the solutions A and B, and as a result, variation of a bicarbonate ion content may not be inhibited.

The vial A to be filled with the solution A is not limited particularly, and a glass vial and a vial made of a resin can be used. In particular, when preparing the standard reagent by mixing the solutions A and B, in the case of mixing by pouring the solution A filled in the vial A into the solution B, an inside of the vial A at least having water-repellency is more preferable, and a water-repellent resin vial is further preferable so that an amount the solution A remaining in the vial is as small as possible. Further, the total light transmittance of the vial A is preferably 85% or more, more preferably 90% or more from the viewpoint that a content therein is visible easily.

A volume of the vial A is not limited particularly as long as the solution A can be filled therein. In the case where at least the inside of the vial A is not water-repellent, it is desirable that the standard reagent is prepared by pouring the solution B filled in the vial B into the vial A, and in that case, it is preferable that the volume of the vial A is not less than a total volume of the solutions A and B, more preferably 1.1 times the total volume of the solutions A and B to enable mixing of them in the vial A.

### <Solution B>

The solution B is an aqueous solution comprising bicarbonate ion and/or carbonate ion. Components other than the electrolyte component, which is capable of reacting with carbonate ion to form a precipitate, may be contained in the solution B. For example, a basic component such as sodium hydroxide can be contained in the solution B to adjust a pH value to be a predetermined one. It is more preferable to combine sodium bicarbonate and sodium carbonate from the viewpoint of easy control of a pH value.

Bicarbonate ion (HCO₃⁻) and carbonate ion (CO₃²⁻) are in an equilibrium state in the aqueous solution. Therefore, in the solution B, either one may be present or both ions may be present together. Example of a preparation method of the solution B include a method of preparing the solution B by adding a pH regulator into sodium bicarbonate or sodium carbonate, and the solution B can also be obtained by dissolving sodium bicarbonate and sodium carbonate at a specific ratio.

A bicarbonate ion concentration in the solution B is preferably 10 mEq/L or more, more preferably 20 mEq/L or more, further preferably 29 mEq/L or more, particularly preferably 40 mEq/L or more, and is preferably 100 mEq/L or less, more preferably 80 mEq/L or less, further preferably 70 mEq/L or less, particularly preferably 50 mEq/L or less. When the bicarbonate ion concentration in the solution B varies, for example, with a lapse of time, a composition of the standard reagent obtained by mixing with the solution A may not be within a desired range, which may make it impossible to use the mixture as a standard reagent. Herein, since bicarbonate ion are present also as carbonate ion reversibly under a basic condition, the bicarbonate ion concentration in the solution B or the standard reagent means a concentration as a sum of bicarbonate ion and carbonate ion.

In the first embodiment of the present invention, the vial B for filling the solution B therein is a water-repellent resin vial. Generally in the case where a liquid being apt to generate carbon dioxide like the solution B is filled in a vial, a glass vial is suitable from the viewpoint that carbon dioxide is hardly penetrated therethrough and a cost is low. However, since the solution B is alkaline, when it is filled in a glass vial, an insoluble matter derived from glass may be generated when the solution B is filled and stored in the vial at high temperature for a long period of time. In the first embodiment of the present invention, concern about generation of such an insoluble matter can be eliminated since the vial B for filling the solution B is formed from a resin.

Also, when preparing the standard reagent, surely mixing the solutions A and B (in other words, mixing a total volume of each solution filled in the vials so that no liquid is left without being mixed) is demanded. In this regard, among vials made of a resin, there is a case where a liquid is left without being mixed depending on kind of a resin. However, in the first embodiment of the present invention, particularly by selecting a water-repellent resin as a resin for forming the vial B, when the solution B filled in the vial B is poured into the vial A, the both solutions can be mixed surely without the solution B being left in the vial B.

An amount of the solution B is preferably 3 mL or more, more preferably 5 mL or more from the viewpoint of controlling a filling amount accurately. Further, the amount of the solution B is preferably 20 mL or less, more preferably 10 mL or less from the viewpoint of handling in mixing operation or the like.

In the first embodiment of the present invention, a pH value of the solution B is preferably 8.9 or more, more preferably 9.2 or more, further preferably 9.3 or more. When the pH value of the solution B is 8.9 or more, there is a tendency that generation of carbon dioxide can be inhibited and storage stability is improved. Further the pH value of the solution B is preferably 10.5 or less, more preferably 10.2 or less, further preferably 10.1 or less from the viewpoint of safety in handling. In adjusting the pH value, while a pH regulator which is generally used in a field of relative art can also be used, it is preferable to use sodium bicarbonate and sodium carbonate and adjust a ratio thereof as mentioned above since preparation operation is easy.

While a volume of the vial B is not limited particularly as long as the solution B can be filled therein, it is preferable that a size of the vial B is large enough to make it possible to mix with the solution in the vial A by one hand. It is preferable to determine the volume of the vial B (whole volume of the vial) in consideration of an amount of the solution B so that a volume of a void space in the vial B is 70% or less of the whole volume of the vial. When the volume of the void space in the vial B becomes large, there is a case where CO2 is apt to be released into the void space from the solution B and a concentration of HCO₃⁻ in the solution decreases. A lower limit of the volume of the void space in the vial B is not limited particularly, and it is better to set the volume to be 20% or more of the whole volume of the vial.

An amount of the standard reagent including the solution A and the solution B after prepared is not limited particularly, and is usually preferably 3 mL or more, more preferably 5mL or more as a minimum amount required for checking a dialysis fluid. Further it is desirable that the standard reagent prepared using the kit according to the first embodiment of the present invention is used up in principle for one use, and from this point of view, an amount of the standard reagent is preferably 50 mL or less, more preferably 30 mL or less.

### <Standard reagent>

When preparing the standard reagent using the standard reagent kit according to the first embodiment of the present invention, it is desirable to mix the solutions by opening the vial A and the vial B, and pouring either of the solution A or the solution B filled in the water-repellent resin vial into the vial filled with another solution. Concretely in the case where the both of the vial A and the vial B are water-repellent resin vials, the solution A may be poured into the vial B, or the solution B may be poured into the vial A. In the case where only the vial B is a water-repellent resin vial, it is preferable to pour the solution B into the vial A. By using such procedures for mixing, the both solutions can be mixed surely without the solution A or B being left in the vial.

It is ideal that the standard reagent for analysis of a dialysis fluid basically has the same composition as that of the target dialysis fluid after prepared. Accordingly, other components to be properly added to the solution A or the solution B in addition to the above-mentioned "bicarbonate ion and/or carbonate ion" in the solution B and "the electrolyte component capable of reacting with carbonate ion to form a precipitate" in the solution A may be determined depending on the target dialysis fluid. Specifically preferable electrolyte compositions of the dialysis fluid (standard reagent) after mixing of the solution A and the solution B are as shown in the following Table 1. One example of an electrolyte composition of the standard reagent, for example, a reagent for powder preparation for a dialysis fluid for an artificial kidney "Lympack (registered trademark) dialysis agent TA3" (manufactured by Nipro Corporation) is as shown in Table 2.

**Table 1**

| Component | Final concentration (mEq/L) |
|---|---|
| Na⁺ | 120 - 160 |
| K⁺ | 0.5 - 3.0 |
| Ca²⁺ | 1.5 - 4.5 |
| Mg²⁺ | 0 - 2.0 |
| Cl⁻ | 90 - 135 |
| CH₃COO⁻ | 0 - 15 |
| HCO₃⁻ | 20 - 35 |
| Glucose | 0 - 2.5 (g/L) |

**Table 2**

| Component | Final concentration (mEq/L) |
|---|---|
| Na⁺ | 140.00 |
| K⁺ | 2.00 |
| Ca²⁺ | 3.00 |
| Mg²⁺ | 1.00 |
| Cl⁻ | 113.0 |
| CH₃COO⁻ | 10.22 |
| HCO₃⁻ | 25.0 |
| Glucose | 1.00 (g/L) |

It is ideal that a pH value of the standard reagent for analysis of a dialysis fluid is at the same level as that of a target dialysis fluid after prepared, and is preferably from 7.0 to 7.5.

### [Standard reagent kit for analysis of a dialysis fluid according to second embodiment]

The standard reagent kit for analysis of a dialysis fluid according to first embodiment of the present invention is a standard reagent kit for analysis of a dialysis fluid comprising (1) a vial A filled with an aqueous solution A comprising an electrolyte component capable of reacting with carbonate ion to form a precipitate, (2) a vial B filled with an aqueous solution B comprising at least either one of bicarbonate ion and carbonate ion, and (3) a transfer needle (dissolution liquid injection needle) for mixing a content of the vial A and a content of the vial B. In the standard reagent kit for analysis of a dialysis fluid according to second embodiment of the present invention, sedimentation of a precipitate is prevented at least for 6 months or more, preferably for one year or more, and a bicarbonate ion concentration and a pH value hardly vary.

### <Solution A>

The solution A is an aqueous solution comprising an electrolyte component capable of reacting with carbonate ion to form a precipitate. Examples of the electrolyte component capable of reacting with carbonate ion to form a precipitate include calcium ion, magnesium ion and the like. These electrolyte components can be contained in the solution A as calcium chloride, magnesium chloride and the like. In the case of using calcium chloride and magnesium chloride for a standard reagent, these are contained in the solution A as electrolytes. It is a matter of course that in the second embodiment of the present invention, carbonate ion and bicarbonate ion are not contained in the solution A.

In addition, it is possible that the solution A contains other electrolytes such as sodium chloride, potassium chloride and sodium acetate and an organic salt. Further, hydrochloric acid and an organic acid can be used as a pH regulator, and it is preferable to use, as an organic acid, acetic acid, citric acid, oxalic acid, tartaric acid, maleic acid, ascorbic acid, oxalacetic acid, gluconic acid, isocitric acid, malic acid, and/or citric acid. Further it is preferable to mix glucose in the solution A.

Examples of usable calcium chloride include calcium chloride dihydrate, calcium chloride monohydrate, calcium chloride anhydride and the like. Examples of magnesium chloride include magnesium chloride hexahydrate and the like.

Anhydrous sodium acetate, sodium acetate trihydrate and the like can be used preferably as sodium acetate. Further, acetic acid (glacial acetic acid) and sodium hydroxide can be added, resulting in addition of sodium acetate.

An amount of the solution A is preferably 3 mL or more, more preferably 5 mL or more from the viewpoint of controlling a filling amount accurately. Further, the amount of the solution A is preferably 20 mL or less, more preferably 10 mL or less from the viewpoint of handling in mixing operation.

A pH value of the solution A is preferably 2.5 or more, more preferably 3 or more in consideration of an influence on corrosion of a production apparatus and the like. Further the pH value of the solution A is preferably 7 or less, more preferably 6 or less. If the pH value of the solution A is too high, a pH value of the solution B cannot help being set to be lower in order to maintain a pH value of the standard reagent obtained by mixing the solutions A and B within a predetermined range, and as a result, variation of a bicarbonate ion content may not be inhibited.

A volume of the vial A is not limited particularly as long as the solution A can be filled therein, and it is preferable that the volume of the vial A is not less than a total volume of the solutions A and B, more preferably 1.1 times the total volume of the solutions A and B to enable mixing of them in the vial A.

### <Solution B>

The solution B is an aqueous solution comprising bicarbonate ion and/or carbonate ion. Components other than the electrolyte components, which are capable of reacting with carbonate ion to form a precipitate, may be contained in the solution B. For example, a basic component such as sodium hydroxide can be contained in the solution B in order to adjust a pH value to be a predetermined one. It is more preferable to combine sodium bicarbonate and sodium carbonate from the viewpoint of easy control of a pH value.

Bicarbonate ion (HCO₃⁻) and carbonate ion (CO₃²⁻) are in an equilibrium state in the aqueous solution. Therefore, in the solution B, either one may be present or both ions may be present together. Example of a preparation method of the solution B include a method of preparing the solution B by adding a pH regulator into sodium bicarbonate or sodium carbonate, and the solution B can also be obtained by dissolving sodium bicarbonate and sodium carbonate at a specific ratio.

A bicarbonate ion concentration in the solution B is preferably 10 mEq/L or more, more preferably 20 mEq/L or more, further preferably 29 mEq/L or more, particularly preferably 40 mEq/L or more, and is preferably 100 mEq/L or less, more preferably 80 mEq/L or less, further preferably 70 mEq/L or less, particularly preferably 50 mEq/L or less. When the bicarbonate ion concentration in the solution B varies, for example, with a lapse of time, a composition of the standard reagent obtained by mixing with the solution A may not be within a desired range, which may make it impossible to use the mixture as a standard reagent. Herein, since bicarbonate ion are present also as carbonate ion reversibly under a basic condition, the bicarbonate ion concentration in the solution B or the standard reagent means a concentration as a sum of bicarbonate ion and carbonate ion.

An amount of the solution B is preferably 3 mL or more, more preferably 5 mL or more from the viewpoint of controlling a filling amount accurately. Further, the amount of the solution B is preferably 20 mL or less, more preferably 10 mL or less from the viewpoint of handling in mixing operation.

In the second embodiment of the present invention, a pH value of the solution B is preferably 8.6 or more, more preferably 9.0 or more, further preferably 9.2 or more. When the pH value of the solution B is 8.6 or more, there is a tendency that storage stability is improved. Further the pH value of the solution B is preferably 10.5 or less, more preferably 10.2 or less, further preferably 10.1 or less from the viewpoint of safety in handling. In adjusting the pH value, while a pH regulator which is generally used in a field of relative art can also be used, it is preferable to use sodium bicarbonate and sodium carbonate and adjust a ratio thereof as mentioned above since preparation operation becomes easy.

While a volume of the vial B is not limited particularly as long as the solution B can be filled therein, it is preferable that a size of the vial B is large enough to make it possible to mix with the solution in the vial A by one hand. It is preferable to determine the volume of the vial B (whole volume of the vial) in consideration of an amount of the solution B so that a volume of a void space in the vial B is 70% or less of the whole volume of the vial. When the volume of the void space in the vial B becomes large, there is a case where CO₂ is apt to be released into the void space from the solution B and a concentration of HCO₃⁻ in the solution decreases. A lower limit of the volume of the void space in the vial B is not limited particularly, and it is better to set the volume to be 20% or more of the whole volume of the vial.

An amount of the standard reagent including the solution A and the solution B after prepared is not limited particularly, and is usually preferably 3 mL or more, more preferably 5mL or more as a minimum amount required for checking a dialysis fluid. Further it is desirable that the standard reagent prepared using the kit according to the second embodiment of the present invention is used up in principle for one use, and from this point of view, an amount of the standard reagent is preferably 50 mL or less, more preferably 30 mL or less.

### <Transfer needle>

The kit according to the second embodiment of the present invention comprises a transfer needle for communicating the vial A containing the solution A and the vial B containing the solution B and mixing the solutions at time of use. The transfer needle is not limited particularly as long as it is a communicating member having hollow needles at both ends thereof for communicating the vial A and the vial B.

### <Standard reagent>

It is ideal that the standard reagent for analysis of a dialysis fluid basically has the same composition as that of the target dialysis fluid after prepared. Accordingly, other components to be properly added to the solution A or the solution B in addition to the above-mentioned "bicarbonate ion and/or carbonate ion" in the solution B and "the electrolyte component capable of reacting with carbonate ion to form a precipitate" in the solution A may be determined depending on the target dialysis fluid. Specifically preferable electrolyte composition of the dialysis fluid (standard reagent) after mixing of the solution A and the solution B are as shown in the above Table 1. One example of an electrolyte composition of the standard reagent, for example, a reagent for powder preparation for a dialysis fluid for an artificial kidney "LYMPACK (registered trademark) dialysis agent TA3" (manufactured by Nipro Corporation) is as shown in the above Table 2.

It is ideal that a pH value of the standard reagent for analysis of a dialysis fluid is at the same level as that of a target dialysis fluid after prepared, and is preferably from 7.0 to 7.5.

### [Aqueous solutions for standard reagent, dialysis fluid and substitution fluid for artificial kidney according to third embodiment]

The aqueous solution for the standard reagent for analysis of a dialysis fluid according to the third embodiment of the present invention, which includes bicarbonate ion and/or carbonate ion, is a preparation including sodium bicarbonate in a sodium bicarbonate-containing dialysis fluid, and is herein referred to as a solution B for the standard reagent or simply a solution B. Further, the aqueous solution for a dialysis fluid according to the third embodiment of the present invention, which includes bicarbonate ion and/or carbonate ion, is a preparation including sodium bicarbonate in a sodium bicarbonate-containing dialysis fluid, and is herein referred to as a concentrated solution including a formulation B for a dialysis fluid or simply a concentrated solution including a formulation B. Furthermore, the aqueous solution for a substitution fluid for an artificial kidney according to the third embodiment of the present invention, which includes bicarbonate ion and/or carbonate ion, is a preparation including sodium bicarbonate in a substitution fluid for an artificial kidney, and is herein referred to as an aqueous solution including a formulation B for a substitution fluid for an artificial kidney or simply an aqueous solution including a formulation B for a substitution fluid.

The solution B for the standard reagent, the concentrated solution including the formulation B for a dialysis fluid and the aqueous solution including the formulation B for a substitution fluid for an artificial kidney according to the third embodiment of the present invention include either one of bicarbonate ion or carbonate ion, and a pH value is from 8.6 to 10.5. The pH value of the solution B, the concentrated solution including the formulation B and the aqueous solution including the formulation B for a substitution fluid is 8.6 or more, preferably 9.0 or more, more preferably 9.2 or more. When the pH value of the solution B, the concentrated solution including the formulation B and the aqueous solution including the formulation B for a substitution fluid is 8.6 or more, variation of the pH value of the solution B, the concentrated solution including the formulation B and the aqueous solution including the formulation B for a substitution fluid, variation of a bicarbonate ion concentration and release of carbon dioxide can be inhibited for a long period of time, and stable solution B, concentrated solution including the formulation B and aqueous solution including the formulation B for a substitution fluid can be obtained. Further, upper limits of the solution B, the concentrated solution including the formulation B and the aqueous solution including the formulation B for a substitution fluid are not limited particularly, and are 10.5 or less, preferably 10.2 or less, more preferably 10.1 or less from the viewpoint of safety in handling, an influence on a production apparatus and the like. In adjusting the pH value, while a pH regulator which is generally used in a field of relative art can also be used, it is preferable to use sodium bicarbonate and sodium carbonate and adjust a ratio thereof since adjusting operation is easy.

Components other than the electrolyte component, which is capable of reacting with carbonate ion to form a precipitate, may be contained in the solution B, the concentrated solution including the formulation B and the aqueous solution including the formulation B for a substitution fluid. For example, a basic component such as sodium hydroxide can be contained in the solution B to adjust a pH value to be a predetermined one. It is more preferable to combine sodium bicarbonate and sodium carbonate from the viewpoint of easy control of a pH value.

The bicarbonate ion concentration in the solution B, the concentrated solution including the formulation B and the aqueous solution including the formulation B for a substitution fluid may be appropriately set depending on a purpose of use thereof and is not limited particularly. Herein, since bicarbonate ion are present also as carbonate ion reversibly under a basic condition, the bicarbonate ion concentration in the solution B, the concentrated solution including the formulation B, the aqueous solution including the formulation B for a substitution fluid, the standard reagent, a dialysis fluid and the substitution fluid for an artificial kidney means a concentration as a sum of bicarbonate ion and carbonate ion.

An amount of the solution B is preferably large enough to make it possible to mix with the solution A by one hand, and for example, is preferably from 2 to 8 mL, more preferably from 4 to 6 mL, most preferably about 5 mL.

It is preferable to fill the solution B in a vial keep it therein. While a volume of the vial is not limited particularly as long as the solution B can be filled therein, it is preferable that a size of the vial is large enough to make it possible to mix with the solution in the vial A by one hand.

It is noted that if a volume of a void space of a vial increases in the light of the vial volume and the filling amount of the solution B, usually there is a tendency that a pH value is apt to increase with a lapse of time. However, in the third embodiment of the present invention, satisfactory stability with time can be secured irrespective of the volume of a void space of a vial by using the pH value of 8.6 or more. Therefore, while the volume of a void space of a vial is not limited particularly, for example, the volume of a void space in the vial is preferably from about 20 to 70% of the whole volume of the vial.

The standard reagent for analysis of a dialysis fluid can be obtained from the solution B according to the third embodiment of the present invention in combination with the aqueous solution for the standard reagent for analysis of a dialysis fluid (herein also referred to as a solution A for the standard reagent or simply a solution A) comprising an electrolyte component capable of reacting with carbonate ion to form a precipitate. Examples of the electrolyte component capable of reacting with carbonate ion to form a precipitate include calcium ion, magnesium ion and the like. These electrolyte components can be contained in the solution A as calcium chloride, magnesium chloride and the like. In the case of using calcium chloride or magnesium chloride, it is contained in the solution A as an electrolyte. It is a matter of course that in the present invention, carbonate ion and bicarbonate ion are not contained in the solution A.

In addition to calcium chloride and magnesium chloride, it is possible that the solution A contains other electrolytes such as sodium chloride, potassium chloride and sodium acetate and an organic salt. Further, hydrochloric acid and an organic acid can be used as a pH regulator, and it is preferable to use, as an organic acid, acetic acid, citric acid, oxalic acid, tartaric acid, maleic acid, ascorbic acid, oxalacetic acid, gluconic acid, isocitric acid, malic acid, and/or citric acid. Further it is preferable to mix glucose in the solution A according to necessity.

Examples of usable calcium chloride include calcium chloride dihydrate, calcium chloride monohydrate, calcium chloride anhydride and the like. Examples of magnesium chloride include magnesium chloride hexahydrate and the like.

Anhydrous sodium acetate, sodium acetate trihydrate and the like are used preferably as sodium acetate. Further, acetic acid (glacial acetic acid) and sodium hydroxide can be added, resulting in addition of sodium acetate.

An amount of the solution A is preferably large enough to make it possible to mix with the solution B by one hand, and for example, is preferably from 2 to 8 mL, more preferably from 4 to 6 mL, most preferably about 5 mL.

A pH value of the solution A is adjusted depending on the pH value of the solution B so that a pH value of the standard reagent obtained by mixing the solution A and the solution B becomes from 7.2 to 7.4, and is preferably 3 or more, more preferably 4 or more in consideration of an influence on corrosion of a production apparatus and the like. Further the pH value of the solution A is preferably 6 or less, more preferably 5 or less. If the pH value of the solution A is too high, a pH value of the solution B cannot help being set to be lower in order to maintain a pH value of the standard reagent obtained by mixing the solutions A and B within a predetermined range, and as a result, variation of a bicarbonate ion content may not be inhibited.

It is preferable that the solution B is also filled in a vial and kept therein. A volume of the vial A is not limited particularly as long as the solution A can be filled therein, and it is preferable that the volume of the vial A is not less than a total volume of the solutions A and B, more preferably 1.1 times the total volume of the solutions A and B to enable mixing of them in the vial A.

An amount of the standard reagent including the solution A and the solution B after prepared is not limited particularly, and is usually preferably 3 mL or more, more preferably 5mL or more as a minimum amount required for checking a dialysis fluid. Further it is desirable that the standard reagent prepared using the aqueous solution according to the third embodiment of the present invention is used up in principle for one use, and from this point of view, an amount of the standard reagent is preferably 50 mL or less, more preferably 30 mL or less.

It is ideal that the standard reagent for analysis of a dialysis fluid basically has the same composition as that of the target dialysis fluid after prepared. Accordingly, other components to be properly added to the solution A or the solution B in addition to the above-mentioned "bicarbonate ion and/or carbonate ion" in the solution B and "the electrolyte component capable of reacting with carbonate ion to form a precipitate" in the solution A may be determined depending on the target dialysis fluid, purpose of dialysis and the like. Specifically preferable electrolyte composition of the dialysis fluid (standard reagent) after mixing of the solution A and the solution B are as shown in the above Table 1. One example of electrolyte composition of the standard reagent, for example, a reagent for powder preparation for a dialysis fluid for an artificial kidney "LYMPACK (registered trademark) dialysis agent TA3" (manufactured by Nipro Corporation) is as shown in the above Table 2.

The standard reagent for analysis of a dialysis fluid according to the third embodiment of the present invention can be obtained, for example, by communicating the vial A containing the solution A and the vial B containing the solution B, for example, by means of a transfer needle and mixing the solution A and the solution B at time of use. The transfer needle is not limited particularly as long as it is a communicating member having hollow needles at both ends thereof for communicating the vial containing the solution A and the vial containing the solution B. Further, the solution A and the solution B may be mixed, for example, by a means for accurately measuring the content of each vial using a hole pipette or the like and mixing the both solutions in a separate vessel. The both solutions A and B contained in the vials may be mixed by pouring a content of one vial to another vial.

It is preferable that the concentrated solution including the formulation B for a dialysis fluid according to the third embodiment of the present invention is usually filled in a vial and kept therein.

A preparation for a sodium bicarbonate-containing dialysis fluid can be obtained from the concentrated solution including the formulation B according to the third embodiment of the present invention in combination with the preparation for a dialysis fluid (herein also referred to as a formulation A for a dialysis fluid or simply a formulation A) comprising an electrolyte component capable of reacting with carbonate ion to form a precipitate. The formulation A may be a solution or a solid preparation, and in the case of a solid preparation, the solid preparation contains, as its salt, the electrolyte component capable of reacting with carbonate ion to form a precipitate, and is formed into an aqueous solution at a time of use and then is combined with the formulation B. Examples of the electrolyte component capable of reacting with carbonate ion to form a precipitate include calcium ion, magnesium ion and the like. These electrolyte components can be contained in the formulation A as calcium chloride, magnesium chloride and the like. In the case of using calcium chloride or magnesium chloride, these are contained in the formulation A as electrolytes. It is a matter of course that in the third embodiment of the present invention, carbonate ion and bicarbonate ion are not contained in the formulation A. In addition, the above-mentioned other electrolytes, an organic salt, glucose and the like exemplified as the components which may be contained in the solution A for the standard reagent can also be contained in the formulation A. The concentrated solution including the formulation B is usually diluted appropriately at a time of use and is combined with the formulation A (dissolved or diluted according to necessity) to prepare a dialysis fluid.

Generally one used as the formulation A for a sodium bicarbonate-containing dialysis fluid can be used as the formulation A by adjusting a pH value thereof appropriately to the pH value of the concentrated solution including the formulation B according to the third embodiment of the present invention. The above mentioned relating the pH value of the solution A for the standard reagent is similarly applied to the pH value of the formulation A. The composition of the electrolyte composition of the dialysis fluid obtained by mixing the formulation A and the concentrated solution including the formulation B may be determined according to a purpose of dialysis, and is preferably the composition shown in the above Table 1, and one example thereof is as shown in Table 2.

The substitution fluid for an artificial kidney can be obtained from the aqueous solution including the formulation B for a substitution fluid according to the third embodiment of the present invention in combination with the preparation for a substitution fluid for an artificial kidney (herein also referred to as a formulation A for a substitution fluid or simply an aqueous solution including a formulation A for a substitution fluid) comprising an electrolyte component capable of reacting with carbonate ion to form a precipitate. Examples of the electrolyte component capable of reacting with carbonate ion to form a precipitate include calcium ion, magnesium ion and the like. These electrolyte components can be contained in the solution A as calcium chloride, magnesium chloride and the like. In the case of using calcium chloride and magnesium chloride, these are contained in the formulation A for a substitution fluid as electrolytes. It is a matter of course that in the third embodiment of the present invention, carbonate ion and bicarbonate ion are not contained in the solution A. In addition, the above-mentioned other electrolytes, an organic salt, glucose and the like exemplified as the components which may be contained in the solution A for the standard reagent can also be contained in the formulation A for a substitution fluid.

The substitution fluid for an artificial kidney is usually of a type for mixing at a time of use and comprises two liquids comprising the aqueous solution including the formulation A for a substitution fluid and the aqueous solution including the formulation B for a substitution fluid. It is preferable that the substitution fluid for an artificial kidney is, for example, a preparation contained in a multi-chamber vessel having at least two chambers which are separated by an isolation means and can be communicated with each other, in which the aqueous solution including the formulation A for a substitution fluid and the aqueous solution including the formulation B for a substitution fluid are put in a first chamber and a second chamber, respectively. A preferred example of such a multi-chamber vessel includes a double bag vessel made of plastic such a polypropylene, and conventional vessel used for a substitution fluid for a filtration type artificial kidney can be used. Also a conventional filling method, a method of use and the like can be applied.

One generally used as a formulation A for a substitution fluid for an artificial kidney can be used as the aqueous solution including the formulation A for a substitution fluid by adjusting a pH value thereof appropriately to the pH value of the aqueous solution including the formulation B for a substitution fluid according to the third embodiment of the present invention. The above mentioned relating the pH value of the solution A for the standard reagent is similarly applied to the pH value of the aqueous solution including the formulation A for a substitution fluid. The composition of the electrolyte components of the substitution fluid obtained by mixing the aqueous solution including the formulation A for a substitution fluid and the aqueous solution including the formulation B for a substitution fluid is preferably the composition shown in the above Table 1, and one example thereof is as shown in Table 2.

In the preparation contained in a multi-chamber vessel according to the third embodiment of the present invention, for example, the aqueous solution including the formulation A for a substitution fluid and the aqueous solution including the formulation B for a substitution fluid are filled in the respective chambers of the multiple-chamber and the respective inlets are sealed. Thereafter, a final product is obtained by performing high pressure steam sterilization treatment in accordance with an indication for a final sterilization method of the Japanese Pharmacopoeia.

It is preferable that the multi-chamber vessel is covered with a gas barrier packaging material in order to avoid contact with outside atmosphere. For the covering, the multi-chamber vessel may be sealed in a container made of a gas barrier packaging material. Also, in the case where the packaging material is a film, the multi-chamber vessel may be sealed with the film. Many materials and products such as an ethylene-vinyl alcohol copolymer film are known as a gas barrier packaging material, and generally available ones on the market can be used.

When covering the multi-chamber vessel, the inside of the outer package is in an oxygen-free state. Therefore, a deoxidant may be filled together inside the outer package, or the inside of the outer package may be replaced by a nitrogen gas or a carbon dioxide gas, and it is preferable to fill a deoxidant. Further it is preferable to introduce an oxygen detecting agent for the purpose of detecting a pin hole of the outer package. Generally available ones on the market can be used as the deoxidant and the oxygen detecting agent.

In the substitution fluid for an artificial kidney according to the third embodiment of the present invention, by adjusting the pH value of the aqueous solution including the formulation B for a substitution fluid to be a predetermined value, countermeasures such as use of an outer package made of the above-mentioned gas barrier packaging material, filling of a carbon dioxide gas generating deoxidant, and substitution of an inside atmosphere with nitrogen gas, carbon dioxide gas or the like are not essential.

The present invention is explained below by means of Examples, but is not intended to be limited to the Examples.

### EXAMPLE

### 1. First Embodiment

### Example 1-1

According to the formulation shown in Table 3, each component of the solution A was mixed and dissolved in injection water, and injection water was added so that the total volume became 100 mL to prepare the solution A. In this case, pH value of the solution A was adjusted to be 4.7 by appropriately mixing hydrochloric acid to the solution A. The obtained solution A was filtered through a membrane filter having a pore size of 0.22 µm, and 5.0 mL of the filtrate was filled in a 10 mL COP resin vial ("PVD-10" (COP vial) manufactured by Daiwa Special Glass Co., Ltd.; water contact angle: 100.8 degrees, total light transmittance: 91.5%). After putting a rubber plug on the vial, the vial was sealed with an aluminum cap to obtain the vial A of the solution A.

According to the formulation shown in Table 4, sodium bicarbonate was dissolved in injection water, and sodium hydroxide was added thereto as 1 mol/L of an aqueous solution for injection to adjust pH value to be 9.1, thus preparing the solution B (the total volume of 100 mL). The obtained solution B was filtered through a membrane filter having a pore size of 0.22 µm, and 5.0 mL of the filtrate was filled in a 10 mL COP resin vial ("PVD-10" (COP vial) manufactured by Daiwa Special Glass Co., Ltd.; water contact angle: 100.8 degrees, total light transmittance: 91.5%). After putting a rubber plug on the vial, the vial was sealed with an aluminum cap to obtain a vial B of the solution B. It is noted that the water contact angle was measured using an automatic water contact angle meter ("DSA100S" manufactured by KRUSS GmbH), and the total light transmittance was measured using a hazemeter ("NDH7000" manufactured by NIPPON DENSHOKU INDUSTRIES CO., LTD.).

**Table 3**

| Components of solution A (g) | Example 1-1 |
|---|---|
| Sodium chloride (NaCl) | 1.22 |
| Potassium chloride (KCl) | 0.030 |
| Calcium chloride (CaCl₂·2H₂O) | 0.044 |
| Magnesium chloride (MgCl₂·6H₂O) | 0.020 |
| (Anhydrous) Sodium acetate (CH₃COONa) | 0.13 |
| Acetic acid (CH₃COOH) | 0.027 |
| Hydrochloric acid (HCl) | proper amount |
| Glucose | 0.20 |
| Injection water | proper amount |
| pH (n=3) | 4.7 |

**Table 4**

| Components of solution B (g) | Example 1-1 |
|---|---|
| Sodium hydroxide (NaOH) | 0.018 |
| Sodium bicarbonate (NaHCO₃) | 0.42 |
| Injection water | proper amount |
| pH (n=3) | 9.1 |

In a kit comprising the obtained vial A and vial B, electrolyte components capable of reacting with carbonate ion to form a precipitation were put in the vial A, and bicarbonate ion and carbonate ion were put in the vial B. Therefore, there is no possibility of the contents in the vial B being subject to precipitation even after storage for a long period of time.

A standard reagent was prepared using this kit. Namely, after removing the rubber plug of the vial B and then the rubber plug of the vial A, the solution A was poured into the vial B. The rubber plug was put on the vial B, and the vial B was subjected to mixing by inverting the vial B two times to prepare the standard reagent. The obtained standard reagent was one having a desired composition.

### Example 1-2: Preparation of standard reagent

Standard reagent kits for analysis of a dialysis fluid comprising the vial A and vial B were obtained in the same manner as in Example 1-1. Three test operators prepared standard reagents by pouring the solution A into the vial B and mixing by inverting the vial B two times in the same manner as in Example 1-1 (n=3 each).

### Test Example 1-1: pH value and concentration of bicarbonate ion (n=3 each)

pH values and concentrations of the standard reagents obtained in Example 1-2 were measured using a blood gas analyzer ("RAPIDLab 348EX" available from Siemens AG). The results are shown in Table 5.

**Table 5**

| | Operator A | | Operator B | | Operator C | |
|---|---|---|---|---|---|---|
| | Average | Min. value to max. value | Average | Min. value to max. value | Average | Min. value to max. value |
| pH value | 7.170 | 7.169 to 7.171 | 7.170 | 7.167 to 7.176 | 7.168 | 7.167 to 7.169 |
| Concentration of bicarbonate ion (mEq/L) | 21.8 | 21.0 to 22.4 | 22.2 | 22.0 to 22.3 | 22.5 | 22.4 to 22.6 |

From the above results, it was confirmed that according to the kit of the present invention, a standard reagent having a stable composition can be obtained by surely mixing two solutions with good repeatability, namely by pouring a solution contained in a water-repellent resin vial into an another vial and mixing by inverting.

### Example 1-3: Preparation of standard reagent

A standard reagent kit for analysis of a dialysis fluid comprising the vial A and vial B was obtained in the same manner as in Example 1-1. After removing the rubber plug of the vial A and then the rubber plug of the vial B, the solution A was poured into the vial B. The rubber plug was put on the vial B, and the vial B was subjected to mixing by inverting the vial B two times to prepare the standard reagent (n=5).

### Test Example 1-2: Remaining amount of solution A

In Example 1-3, when producing the kit, a mass of an empty vial A was measured beforehand, and then a mass of the vial A filled with the solution A was measured and an amount (g) of the solution filled therein was calculated. Thereafter, when preparing the standard reagent, after pouring the solution A into the vial B, a mass of the vial A was measured, and the mass of the empty vial A measured beforehand was subtracted therefrom to calculate an amount (g) of the remaining solution, which was then divided by a density of the solution A to calculate a remaining amount (mL) of the solution A. Further, a ratio (percentage) of the remaining amount to the amount of the filled solution was assumed to be a ratio (%) of the remaining amount of the solution A. The results are shown in Table 6.

**Table 6**

| | Test Example 1-2 (n=5) | |
|---|---|---|
| | Average | Min. value to max. value |
| Remaining amount of solution A (mL) | 0.011 | 0.004 to 0.016 |
| Ratio of remaining amount of the solution A (%) | 0.22 | 0.08 to 0.32 |

### Example 1-4: Preparation of standard reagent

A standard reagent kit for analysis of a dialysis fluid comprising a vial A and a vial B was obtained in the same manner as in Example 1-1. After removing a rubber plug of the vial B and then a rubber plug of the vial A, a solution B was poured into the vial A. The rubber plug was put on the vial A, and the vial A was subjected to mixing by inverting the vial A two times to prepare the standard reagent (n=5).

### Comparative Example 1-1: Preparation of standard reagent

A standard reagent kit for analysis of a dialysis fluid comprising a vial A and a vial B was obtained in the same manner as in Example 1-4 except that a glass vial ("borosilicate vial" (10 mL) manufactured by Maruemu Corporation) was used as the vial B instead of the COP resin vial. After removing a rubber plug of the vial B and then the rubber plug of the vial A, the solution B was poured into the vial A. The rubber plug was put on the vial A, and the vial A was subjected to mixing by inverting the vial A two times to prepare the standard reagent (n=5).

### Test Example 1-3: Remaining amount of solution B

In Example 1-4 and Comparative Example 1-1, when producing a kit, a mass of an empty vial B was measured beforehand, and then a mass of the vial B filled with a solution B was measured and an amount (g) of the solution filled therein was calculated. Thereafter, when preparing a standard reagent, after pouring the solution B into the vial A, a mass of the vial B was measured, and the mass of the empty vial B measured beforehand was subtracted therefrom to calculate an amount (g) of the remaining solution, which was then divided by a density of the solution B to calculate a remaining amount (mL) of solution B. Further, a ratio (percentage) of a remaining amount to the amount of filled solution was assumed to be a ratio (%) of a remaining amount of the solution B. The results are shown in Table 7.

**Table 7**

| | Example 1-4 (n=5) | | Comparative Example 1-1 (n=5) | |
|---|---|---|---|---|
| | Average | Min. value to max. value | Average | Min. value to max. value |
| Remaining amount (mL) of solution B | 0.037 | 0.032 to 0.054 | 0.121 | 0.094 to 0.142 |
| Ratio (%) of remaining amount of the solution B | 0.73 | 0.63 to 1.06 | 2.38 | 1.86 to 2.80 |

### Reference Example 1-1

0.42 Gram of sodium bicarbonate was added to 90 mL of injection water and dissolved therein, and injection water was added so that the total volume became 100 mL to prepare a solution B. The obtained solution B was filtered through a membrane filter having a pore size of 0.22 µm, and 5.0 mL of the filtrate was filled in a 10 mL COP resin vial having the same volume as in Example 1-1. After putting a rubber plug on the vial, the vial was sealed with an aluminum cap to obtain the vial B of the solution B. It is noted that a ratio of a void space in the obtained vial B was 65%.

### Reference Example 1-2

A vial B was obtained in the same manner as in Reference Example 1-1 except that 0.42 g of sodium bicarbonate was added to 90 mL of injection water and dissolved therein, 1 mol/L of an aqueous solution of sodium hydroxide for injection was added thereto to adjust pH value to be 8.6, and injection water was added thereto so as to obtain a total volume of 100 mL, thus preparing the solution B.

### Reference Examples 1-3 to 1-8

A vial B was obtained in the same manner as in Reference Example 1-1 except that a proper amount of a 0.1 mol/L aqueous solution of sodium hydroxide for injection was added to 90 mL of injection water, thereafter 0.42 g of sodium bicarbonate was added thereto and dissolved therein, then 0.1 mol/L and 1 mol/L aqueous solutions of sodium hydroxide for injection were added properly thereto to adjust pH value as shown in Table 8, and injection water was added so as to obtain a total volume of 100 mL, thus preparing a solution B.

### Test Example 1-4: Stability test of solution B

A pH value, a carbon dioxide concentration in a void space and a bicarbonate concentration in a void space were measured 10 days (during which the vials were stored at room temperature) after production for the vials B obtained in Reference Examples 1-1 to 1-7, and 9 days (during which the vials were stored at room temperature) after production for the vials B obtained in Reference Example 1-8 (measured values obtained at this point of time are indicated in columns of "at start" in Table 8). Thereafter, each vial was stored at 60°C, and a pH value, a carbon dioxide concentration in a void space and a bicarbonate concentration in a void space of each vial were measured again 10 days and 21 days after starting storage thereof at 60°C. A method of each measurement is shown below. The results are shown in Table 8.

### (A) Measurement of pH value (n=3 each)

Two milliliters of a solution in the vial was put in a test tube and a pH value thereof was measured with a pH meter.

### (B) Carbon dioxide concentration in void space (n=3 each)

0.1 Milliliter of a gas in a void space of the vial was subjected to analysis by gas chromatography to obtain a proportion (%) of carbon dioxide in the void space. It is noted that an initial carbon dioxide concentration in the void space is 0.04% similarly to the carbon dioxide concentration in the atmosphere. The results are shown in Table 8.

### (C) Bicarbonate ion concentration (n=3 each)

Two milliliters of a solution in the vial was subjected to analysis by high-performance liquid chromatography (HPLC) under the following conditions to measure a bicarbonate ion concentration. HPLC apparatus: "LC20AD" manufactured by Shimadzu Corporation Column: "IC SI-90 4E" manufactured by Showa Denko K.K. (particle size: 9 µm, inner diameter: 4 mm, diameter: 250 mm manufactured by Showa Denko K.K.)
Moving phase: A solution obtained by mixing 0.371 g of boric acid, 3.28 g of D-mannitol and 1.15 g of tris(hydroxymethyl)aminomethane in 1 liter of the aqueous solution.

**Table 8**

| | pH | pH (n=3) | | | CO₂ (%) (n=3) | | | Bicarbonate ion concentration* (% of labeled amount (n=3) | | |
|---|---|---|---|---|---|---|---|---|---|---|
| | At prepared | At start (room temp.) | 10 days after (60°C) | 21 days after (60°C) | At start (room temp.) | 10 days after (60°C) | 21 days after (60°C) | At start (room temp.) | 10 days after (60°C | 21 days after (60°C) |
| Ref. Ex. 1-1 | 8.3 | 8.57 (0.01) | 8.64 (0.01) | 8.68 (0.01) | 0.475 | 0.393 | 0.363 | 98.21 (0.40) | 97.21 (0.48) | 97.82 (0.28) |
| Ref. Ex. 1-2 | 8.6 | 8.75 (0.01) | 8.79 (0.01) | 8.81 (0.00) | 0.296 | 0.268 | 0.251 | 98.44 (0.29) | 97.78 (0.31) | 97.48 (0.70) |
| Ref. Ex. 1-3 | 8.9 | 8.90 (0.01) | 8.92 (0.02) | 8.93 (0.01) | 0.204 | 0.193 | 0.192 | 99.26 (0.50) | 98.69 (0.27) | 97.92 (0.47) |
| Ref. Ex. 1-4 | 9.0 | 8.96 (0.01) | 8.97 (0.01) | 9.00 (0.02) | 0.175 | 0.166 | 0.172 | 100.73 (0.92) | 100.49 (0.77) | 99.68 (0.89) |
| Ref. Ex. 1-5 | 9.2 | 9.15 (0.02) | 9.15 (0.00) | 9.14 (0.00) | 0.113 | 0.112 | 0.123 | 102.15 (0.42) | 101.04 (0.19) | 100.78 (0.42) |
| Ref. Ex. 1-6 | 9.3 | 9.28 (0.01) | 9.29 (0.01) | 9.29 (0.00) | 0.075 | 0.078 | 0.079 | 100.47 (0.63) | 100.67 (1.10) | 100.66 (1.00) |
| Ref. Ex. 1-7 | 9.5 | 9.45 (0.01) | 9.45 (0.00) | 9.45 (0.01) | 0.053 | 0.051 | 0.055 | 102.01 (0.15) | 102.29 (0.41) | 101.89 (0.44) |
| Ref. Ex. 1-8 | 9.8 | 9.76 (0.01) | 9.76 (0.01) | 9.77 (0.01) | 0.000 | 0.000 | 0.000 | 101.75 (0.23) | 101.46 (0.23) | 101.98 (0.22) |

| | | | | | | | | | | |
|---|---|---|---|---|---|---|---|---|---|---|
| *: Bicarbonate ion exist as carbonate ion reversibly under basic condition, and therefore "Bicarbonate ion concentration" means a sum of bicarbonate ion and carbonate ion. A labeled amount is 50 mmol/L Numerical values in parentheses: S.D. | | | | | | | | | | |

From the results of this accelerated test, it can be presumed that when the solution B containing no electrolyte component capable of reacting with carbonate ion to form a precipitate and comprises at least either one of carbonate ion and bicarbonate ion is filled and stored in a water-repellent resin vial, release of carbon dioxide into the void space is inhibited, variation with time of a pH value and a bicarbonate ion concentration is small and a pH value and a bicarbonate ion concentration are stable for a long period of time. In particular, in Reference Examples 1-3 to 1-8, where a pH value is 8.9 or more, it was seen that during a period of time from the preparation to the starting of the measurement where the solution was stored at room temperature, release of carbon dioxide into the void space can be inhibited more surely, variation with time of a pH value and a bicarbonate ion concentration can be inhibited more surely, and high stability of a pH value and a bicarbonate ion concentration can be obtained over a long period of time.

### 2. Second embodiment

### Example 2-1

According to the same formulation as in Example 1-1 shown in Table 3, each component of the solution A was mixed and dissolved in injection water, and injection water was added so that the total volume became 100 mL to prepare the solution A. In this case, a pH value of the solution A was adjusted to be 4.7 by appropriately mixing hydrochloric acid to the solution A. The obtained solution A was filled in a vial, 5 mL at a time, and after putting a rubber plug on the vial, the vial was sealed by winding an aluminum seal to obtain the vial A of the solution A.

According to the same formulation as in Example 1-1 shown in Table 4, sodium bicarbonate was dissolved in injection water, and sodium hydroxide was added thereto to adjust a pH value to be 9.1, thus preparing a solution B (a total volume of 100 mL). The obtained solution B was filled in a vial, 5 mL at a time, and after putting a rubber plug on the vial, the vial was sealed by winding an aluminum seal to obtain a vial B of the solution B. In this case, a volume of a void space in the vial of the solution B was 65% of the whole volume of the vial.

In a kit comprising the obtained vial A, the obtained vial B and a transfer needle (manufactured by Nipro Corporation), the electrolyte component capable of reacting with carbonate ion to form a precipitate was put in the vial A, and bicarbonate ion and carbonate ion were put in the vial B. Therefore, there is no possibility of the contents in the vial B being subject to precipitation even after storage for a long period of time.

A standard reagent was prepared using this kit. Namely, the vial A and the vial B were communicated with each other by means of the transfer needle and the solutions were mixed to obtain a standard reagent. It was confirmed that a pH value of the solution mixture (n=3) comprising the solution A and the solution B was 7.3 which was within a target pH value (7.2 to 7.4) without a problem. The composition of the obtained standard reagent is shown in Table 9.

**Table 9**

| Components | Final concentration (mEq/L) |
|---|---|
| Na⁺ | 140.00 |
| K⁺ | 2.00 |
| Ca²⁺ | 3.00 |
| Mg²⁺ | 1.00 |
| Cl⁻ | 113.0 |
| CH₃COO⁻ | 10.22 |
| HCO₃⁻ | 25.0 |
| Glucose | 1.00 (g/L) |
| pH value (n=3) | 7.3 |

### Reference Examples 2-1 to 2-10

According to the formulation shown in Table 10, sodium bicarbonate was dissolved in injection water and sodium hydroxide was added thereto to adjust a pH value to be a predetermined one shown in Table 10 to prepare a solution B. The obtained solution B was filled in a vial, 5 mL at a time, and after putting a rubber plug on the vial, the vial was sealed by winding an aluminum seal to obtain a vial B of the solution B. In this case, a volume of a void space in the vial of the solution B was 43% of the whole volume of the vial. It is noted that in Reference Example 2-1, sodium hydroxide was not added.

**Table 10**

| Components of solution B per vial (mg) | Reference Example | | | | | | | | | |
|---|---|---|---|---|---|---|---|---|---|---|
| | 2-1 | 2-2 | 2-3 | 2-4 | 2-5 | 2-6 | 2-7 | 2-8 | 2-9 | 2-10 |
| Sodium bicarbonate (NaHCO₃) | 21 | 21 | 21 | 21 | 21 | 21 | 21 | 21 | 21 | 21 |
| Sodium hydroxide (NaOH) | - | proper amount | proper amount | proper amount | proper amount | proper amount | proper amount | proper amount | proper amount | proper amount |
| Injection water | proper amount | proper amount | proper amount | proper amount | proper amount | proper amount | proper amount | proper amount | proper amount | proper amount |
| pH value | 8.3 | 8.5 | 8.7 | 8.9 | 9.1 | 9.3 | 9.5 | 9.7 | 9.9 | 10.1 |
| Sum of bicarbonate ion and carbonate ion (mmol/L)* | 50 | 50 | 50 | 50 | 50 | 50 | 50 | 50 | 50 | 50 |
| Amount of liquid filled (mL) | 5 | 5 | 5 | 5 | 5 | 5 | 5 | 5 | 5 | 5 |
| Volume of void space (%) | 43 | 43 | 43 | 43 | 43 | 43 | 43 | 43 | 43 | 43 |

| | | | | | | | | | | |
|---|---|---|---|---|---|---|---|---|---|---|
| *: Bicarbonate ion exist as carbonate ion reversibly under basic condition, and therefore "Bicarbonate ion concentration" means a sum of bicarbonate ion and carbonate ion. | | | | | | | | | | |

### Test Example 2-1: Stability test of solution B

A pH value and a carbon dioxide concentration in a void space were measured 2 days (during which the vials were stored at room temperature) after preparation for the vials obtained in Reference Examples 2-1 to 2-10. Thereafter, each vial was stored at 40°C and a pH value and a carbon dioxide concentration in a void space were measured again 2 weeks and 4 weeks after starting storage thereof at 40°C. A method of each measurement is shown below.

### (A) Measurement of pH value (n=3 each)

Two milliliters of a solution in the vial was put in a test tube and a pH value thereof was measured with a pH meter. The results are shown in Table 11.

### (B) Carbon dioxide concentration in void space (n=3 each)

0.1 Milliliter of a gas in a void space of the vial was subjected to analysis by gas chromatography to obtain a proportion (%) of carbon dioxide in the void space. It is noted that an initial carbon dioxide concentration in the void space is 0.04% similarly to the carbon dioxide concentration in the atmosphere. The results are shown in Table 11.

**Table 11**

| | pH (n=3) | | | | CO₂ (%) (n=3) | | |
|---|---|---|---|---|---|---|---|
| | Set value | 2 days after (room temp.) | 2 weeks after (40°C) | 4 weeks after (40°C) | 2 days after (room temp.) | 2 weeks after (40°C) | 4 weeks after (40°C) |
| Ref. Ex. 2-1 | 8.3 | 8.49 | 8.51 | 8.52 | 0.61 | 0.57 | 0.56 |
| Ref. Ex. 2-2 | 8.5 | 8.62 | 8.63 | 8.63 | 0.42 | 0.42 | 0.41 |
| Ref. Ex. 2-3 | 8.7 | 8.76 | 8.76 | 8.77 | 0.29 | 0.30 | 0.29 |
| Ref. Ex. 2-4 | 8.9 | 8.93 | 8.93 | 8.92 | 0.19 | 0.20 | 0.20 |
| Ref. Ex. 2-5 | 9.1 | 9.12 | 9.11 | 9.10 | 0.12 | 0.13 | 0.13 |
| Ref. Ex. 2-6 | 9.3 | 9.31 | 9.30 | 9.29 | 0.08 | 0.08 | 0.07 |
| Ref. Ex. 2-7 | 9.5 | 9.51 | 9.48 | 9.48 | 0.04 | 0.05 | 0.04 |
| Ref. Ex. 2-8 | 9.7 | 9.70 | 9.68 | 9.68 | 0.02 | 0.03 | 0.03 |
| Ref. Ex. 2-9 | 9.9 | 9.91 | 9.88 | 9.87 | 0.01 | 0.01 | 0.01 |
| Ref. Ex. 2-10 | 10.1 | 10.10 | 10.08 | 10.06 | 0.01 | 0.01 | 0.01 |

From the results of this accelerated test, it can be presumed that in the solution B containing no electrolyte component capable of reacting with carbonate ion to form a precipitate and comprising at least either one of carbonate ion and bicarbonate ion, in all of Reference Examples 2-1 to 2-10, in particular, in Reference Examples 2-3 to 2-10, where a pH value was 8.6 or more, release of carbon dioxide into the void space can be inhibited, variation of a pH value is small, and stability of a pH value can be obtained over a long period of time. Further from this it can be said that variation of bicarbonate ion concentration is also small.

### 3. Third embodiment

### Examples 3-1 to 3-8 and Comparative Example 3-1 to 3-2

According to the formulation shown in Table 12, sodium bicarbonate was dissolved in injection water and sodium hydroxide was added thereto to adjust a pH value to be a predetermined one shown in Table 12 to prepare a solution B. The obtained solution B was filled in a vial, 5 mL at a time, and after putting a rubber plug on the vial, the vial was sealed by winding an aluminum seal to obtain a vial of the solution B. In this case, a volume of a void space in the vial was 43% of the whole volume of the vial. It is noted that in Comparative Example 3-1, sodium hydroxide was not used.

**Table 12**

| Components of solution B per vial (mg) | Example | | | | | | | | Com. Ex. | |
|---|---|---|---|---|---|---|---|---|---|---|
| | 3-1 | 3-2 | 3-3 | 3-4 | 3-5 | 3-6 | 3-7 | 3-8 | 3-1 | 3-2 |
| Sodium bicarbonate (NaHCO₃) | 21 | 21 | 21 | 21 | 21 | 21 | 21 | 21 | 21 | 21 |
| Sodium hydroxide (NaOH) | proper amount | proper amount | proper amount | proper amount | proper amount | proper amount | proper amount | proper amount | - | proper amount |
| Injection water | proper amount | proper amount | proper amount | proper amount | proper amount | proper amount | proper amount | proper amount | proper amount | proper amount |
| pH value | 8.7 | 8.9 | 9.1 | 9.3 | 9.5 | 9.7 | 9.9 | 10.1 | 8.3 | 8.5 |
| Sum of bicarbonate ion and carbonate ion (mmol/L)* | 50 | 50 | 50 | 50 | 50 | 50 | 50 | 50 | 50 | 50 |
| Amount of liquid filled (mL) | 5 | 5 | 5 | 5 | 5 | 5 | 5 | 5 | 5 | 5 |
| Volume of void space (%) | 43 | 43 | 43 | 43 | 43 | 43 | 43 | 43 | 43 | 43 |

| | | | | | | | | | | |
|---|---|---|---|---|---|---|---|---|---|---|
| *: Bicarbonate ion exist as carbonate ion reversibly under basic condition, and therefore "Bicarbonate ion concentration" means a sum of bicarbonate ion and carbonate ion. | | | | | | | | | | |

### Examples 3-9 to 3-12 and Comparative Example 3-3 to 3-6

According to the formulation shown in Table 13, vials B of the solution B were obtained in the same manner as in Example 3-1 except that bicarbonate ion concentration was increased by using sodium carbonate instead of sodium hydroxide or a volume of a void space was made small by changing a volume of a vial and an amount of a filling liquid. It is noted that in Comparative Examples 3-3 to 3-6, sodium carbonate was not used.

**Table 13**

| Components of solution B per vial (mg) | Example | | | | Comparative Example | | | |
|---|---|---|---|---|---|---|---|---|
| | 3-9 | 3-10 | 3-11 | 3-12 | 3-3 | 3-4 | 3-5 | 3-6 |
| Sodium bicarbonate (NaHCO₃) | 21 | 42 | 10.5 | 21 | 42 | 84 | 21 | 42 |
| Sodium carbonate (Na₂CO₃) | 26.5 | 53 | 13.25 | 26.5 | - | - | - | - |
| Injection water | proper amount | proper amount | proper amount | proper amount | proper amount | proper amount | proper amount | proper amount |
| pH value | 10.0 | 9.9 | 10.0 | 9.9 | 8.4 | 8.3 | 8.4 | 8.3 |
| Sum of bicarbonate ion and carbonate ion (mmol/L)* | 50 | 100 | 50 | 100 | 50 | 100 | 50 | 100 |
| Amount of liquid filled (mL) | 10 | 10 | 5 | 5 | 10 | 10 | 5 | 5 |
| Volume of void space (%) | 28 | 28 | 43 | 43 | 28 | 28 | 43 | 43 |

| | | | | | | | | |
|---|---|---|---|---|---|---|---|---|
| *: Bicarbonate ion exist as carbonate ion reversibly under basic condition, and therefore "Bicarbonate ion concentration" means a sum of bicarbonate ion and carbonate ion. | | | | | | | | |

### Test Example 3-1: Stability test of solution B

A pH value and a carbon dioxide concentration in a void space were measured 2 days (during which the vials were stored at room temperature) after preparation for the vials obtained in Examples 3-1 to 3-8 and Comparative Examples 3-1 and 3-2, and 17 days (during which the vials were stored at room temperature) after preparation for the vials obtained in Examples 3-9 to 3-12 and Comparative Examples 3-3 to 3-6. Thereafter, each vial was stored at 40°C, and a pH value and a carbon dioxide concentration in a void space were measured again 2 weeks and 4 weeks after starting storage thereof at 40°C. A method of each measurement is shown below.

### (A) Measurement of pH value (n=3 each)

Two milliliters of a solution in the vial was put in a test tube and a pH value thereof was measured with a pH meter. The results are shown in Table 14.

### (B) Carbon dioxide concentration in void space (n=3 each)

0.1 Milliliter of a gas in a void space of the vial was subjected to analysis by gas chromatography to obtain a proportion (%) of carbon dioxide in the void space. It is noted that an initial carbon dioxide concentration in the void space is 0.04% similarly to the carbon dioxide concentration in the atmosphere. The results are shown in Table 14.

**Table 14**

| | pH (n=3) | | | | CO₂ (%) (n=3) | | |
|---|---|---|---|---|---|---|---|
| | Set value | 2 days or 17 days after (room temp.) | 2 weeks after (40°C) | 4 weeks after (40°C) | 2 days or 17 days after (room temp.) | 2 weeks after (40°C) | 4 weeks after (40°C) |
| Ex. 3-1 | 8.7 | 8.76 | 8.76 | 8.77 | 0.29 | 0.30 | 0.29 |
| Ex. 3-2 | 8.9 | 8.93 | 8.93 | 8.92 | 0.19 | 0.20 | 0.20 |
| Ex. 3-3 | 9.1 | 9.12 | 9.11 | 9.10 | 0.12 | 0.13 | 0.13 |
| Ex. 3-4 | 9.3 | 9.31 | 9.30 | 9.29 | 0.08 | 0.08 | 0.07 |
| Ex. 3-5 | 9.5 | 9.51 | 9.48 | 9.48 | 0.04 | 0.05 | 0.04 |
| Ex. 3-6 | 9.7 | 9.70 | 9.68 | 9.68 | 0.02 | 0.03 | 0.03 |
| Ex. 3-7 | 9.9 | 9.91 | 9.88 | 9.87 | 0.01 | 0.01 | 0.01 |
| Ex. 3-8 | 10.1 | 10.10 | 10.08 | 10.06 | 0.01 | 0.01 | 0.01 |
| Ex. 3-9 | 10.0 | 10.04 | 10.06 | 10.04 | 0.01 | 0.01 | 0.01 |
| Ex. 3-10 | 9.9 | 9.96 | 9.97 | 9.95 | 0.02 | 0.02 | 0.02 |
| Ex. 3-11 | 10.0 | 10.04 | 10.05 | 10.04 | 0.01 | 0.01 | 0.01 |
| Ex. 3-12 | 9.9 | 9.96 | 9.96 | 9.95 | 0.02 | 0.02 | 0.02 |
| Com. Ex. 3-1 | 8.3 | 8.49 | 8.51 | 8.52 | 0.61 | 0.57 | 0.56 |
| Com. Ex. 3-2 | 8.5 | 8.62 | 8.63 | 8.63 | 0.42 | 0.42 | 0.41 |
| Com. Ex. 3-3 | 8.4 | 8.61 | 8.69 | 8.77 | 0.43 | 0.40 | 0.31 |
| Com. Ex. 3-4 | 8.3 | 8.51 | 8.57 | 8.64 | 1.10 | 0.97 | 0.77 |
| Com. Ex. 3-5 | 8.4 | 8.70 | 8.78 | 8.87 | 0.37 | 0.30 | 0.24 |
| Com. Ex. 3-6 | 8.3 | 8.58 | 8.66 | 8.75 | 0.99 | 0.80 | 0.59 |

From the above-mentioned results, it was confirmed that while two to 17 days after the preparation in Comparative Examples 3-1 to 3-6, increase in a pH value of 0.1 or more from the pH value at the time of preparation was recognized, in any of Examples 3-1 to 3-12, variation of a pH value after the preparation was small and almost no variation with time of a pH value under high temperature condition was recognized. Further, while it was recognized that in Comparative Examples 3-3 to 3-6, in which a pH value was not within a predetermined range, there was a tendency that variation of a pH value after the preparation was significantly high when a volume of a void space of a vial was increased (Comparative Example 3-3 vs Comparative Example 3-5, Comparative Example 3-4 vs Comparative Example 3-6), it was confirmed that in Examples 3-9 to 3-12, there was almost no influence of the volume of the void space and variation of a pH value after the preparation was inhibited even if the volume of the void space was increased (Example 3-9 vs Example 3-11, Example 3-10 vs Example 3-12).

### Example 3-13

According to the same formulation as in Example 1-1 shown in Table 3, components of a solution A was mixed and dissolved in injection water, and injection water was added thereto so that a total volume became 100 mL to prepare the solution A. In this case, a pH value of the solution A was adjusted to be 4.7 by appropriately mixing hydrochloric acid to the solution A. The obtained solution A was filled in a vial, 5 mL at a time to obtain a vial of the solution A.

Similarly, according to the same formulation as in Example 1-4 shown in Table 4, components of a solution B was dissolved in injection water, and injection water was added thereto so that a total volume became 100 mL to prepare the solution B. The obtained solution B was filled, 5 mL at a time, in a vial having a volume of 10 mL to obtain a vial of the solution B. A volume of a void space in the vial of the solution B was 65% of the whole volume of the vial.

The obtained vial of the solution A and vial of the solution B were communicated with each other by means of the transfer needle (manufactured by Nipro Corporation) and the solutions were mixed to obtain a standard reagent. It was confirmed that a pH value of the solution mixture (n=3) comprising the solution A and the solution B was 7.3 which was within a target pH value (7.2 to 7.4) without a problem. The composition of the obtained standard reagent is shown in Table 15.

**Table 15**

| Components | Final concentration (mEq/L) |
|---|---|
| Na⁺ | 140.00 |
| K⁺ | 2.00 |
| Ca²⁺ | 3.00 |
| Mg²⁺ | 1.00 |
| Cl⁻ | 113.0 |
| CH₃COO⁻ | 10.22 |
| HCO₃⁻ | 25.0 |
| Glucose | 1.00 (g/L) |
| pH value (n=3) | 7.3 |

## Claims

1. A standard reagent kit for analysis of a dialysis fluid comprising:
(1) a vial A filled with an aqueous solution A comprising an electrolyte component capable of reacting with carbonate ion to form a precipitate, and
(2) a vial B filled with an aqueous solution B comprising at least either one of bicarbonate ion and carbonate ion,
wherein at least the vial B is a water-repellent resin vial.

2. The standard reagent kit of claim 1, wherein a contact angle of water on a surface of the water-repellent resin vial is 90 degrees or more.

3. The standard reagent kit of claim 1 or 2, wherein a total light transmittance of the water-repellent resin vial is 85% or more.

4. The standard reagent kit of any one of claims 1 to 3, wherein the water-repellent resin vial is formed from a cycloolefin polymer (COP).

5. The standard reagent kit of any one of claims 1 to 4, wherein a pH value of the solution B is 8.9 or more.

6. The standard reagent kit of any one of claims 1 to 5, wherein the standard reagent for analysis of a dialysis fluid is prepared by pouring either one of the solution A or the solution B filled in the water-repellent resin vial into a vial filled with another solution and subjecting the solutions to mixing.

7. A standard reagent kit for analysis of a dialysis fluid comprising:
(1) a vial A filled with an aqueous solution A comprising an electrolyte component capable of reacting with carbonate ion to form a precipitate,
(2) a vial B filled with an aqueous solution B comprising at least either one of bicarbonate ion and carbonate ion, and
(3) a transfer needle for mixing a content of the vial A and a content of the vial B.

8. The standard reagent kit of claim 7, wherein a pH value of the solution B is from 8.6 to 10.5.

9. The standard reagent kit of claim 7 or 8, wherein the electrolyte component capable of reacting with carbonate ion to form a precipitate comprise a calcium ion and a magnesium ion.

10. An aqueous solution comprising at least either one of bicarbonate ion and carbonate ion and having a pH value of from 8.6 to 10.5, wherein the aqueous solution is combined with an aqueous solution comprising an electrolyte component capable of reacting with carbonate ion to form a precipitate and is used as a standard reagent for analysis of a dialysis fluid.

11. The aqueous solution of claim 10 filled in a vial.

12. A standard reagent for analysis of a dialysis fluid, the standard reagent being prepared by combining an aqueous solution comprising an electrolyte component capable of reacting with carbonate ion to form a precipitate with the aqueous solution of claim 10 or 11.

13. An aqueous solution comprising at least either one of bicarbonate ion and carbonate ion and having a pH value of from 8.6 to 10.5, wherein the aqueous solution is combined with an aqueous solution comprising an electrolyte component capable of reacting with carbonate ion to form a precipitate and is used as a dialysis fluid.

14. An aqueous solution comprising at least either one of bicarbonate ion and carbonate ion and having a pH value of from 8.6 to 10.5, wherein the aqueous solution is combined with an aqueous solution comprising an electrolyte component capable of reacting with carbonate ion to form a precipitate and is used as a substitution fluid for an artificial kidney.

15. The aqueous solution of claim 14 filled in one chamber of a multi-chamber vessel having at least two chambers which are separated by an isolation means and can be communicated with each other.

16. A substitution fluid for an artificial kidney prepared by combining an aqueous solution comprising an electrolyte component capable of reacting with carbonate ion to form a precipitate with the aqueous solution of claim 14 or 15.
